# EUROPÄISCHE PATENTSCHRIFT

(11) **EP 2 296 958 B1**
(45) Veröffentlichungstag und Bekanntmachung des Hinweises auf die Patenterteilung: **02.01.2013**
(21) Anmeldenummer: 09765601.1
(22) Anmeldetag: 16.06.2009
(51) Int. Cl.: B62D 25/16, B62D 21/15, B60R 21/34

(54) **ABSORBERELEMENT FÜR EIN FAHRZEUG**
ABSORBER ELEMENT FOR A VEHICLE
ELEMENT ABSORBEUR POUR UN VEHICULE

(30) Priorität: 18.06.2008 DE 202008008071 U
(43) Veröffentlichungstag der Anmeldung: 23.03.2011
(73) Patentinhaber: Rehau AG + Co, 95111 Rehau (DE)
(72) Erfinder: LUX, Stefan, 95182 Döhlau (DE); TUCHBREITER, Tobias, 95482 Gefrees (DE); HERRMANN, Stefan, 95111 Rehau (DE); SPERRER, Carsten, 95689 Fuchsmühl (DE)
(86) Internationale Anmeldenummer: PCT/EP2009/004324
(87) Internationale Veröffentlichungsnummer: WO 2009/153021

(56) Entgegenhaltungen:
- DE-A1- 10 355 735
- FR-A1- 2 860 763
- FR-A1- 2 902 390
- GB-A- 1 464 031
- JP-A- 2006 044 312
- US-A- 3 809 420

## Beschreibung

Die Erfindung betrifft ein Absorberelement für ein Fahrzeug sowie ein mit dem erfindungsgemäßen Absorberelement ausgestattetes Fahrzeug. Derartige Absorberelemente werden dazu verwendet, im Außenbereich von Fahrzeugen, insbesondere von Kraftfahrzeugen, auftretende Stöße durch Fremdkörper abzumildern. Eine wesentliche Anwendung der genannten Absorberelemente liegt dabei im Bereich des Fußgängerschutzes. Gerade Fußgänger sind bei einer Kollision mit Kraftfahrzeugen im Straßenverkehr ganz erheblichen Gefahren ausgesetzt. Oftmals wird ein Fußgänger bei einem Frontalzusammenstoß mit einem Fahrzeug durch die Frontpartie des Fahrzeugs von den Beinen gerissen und prallt mit seinem Kopf im Bereich der Motorhaube oder im oberen Kotflügelbereich des Fahrzeugs auf. Um die Schwere der hierdurch zu erwartenden Versetzungen abzumildern, ist es wünschenswert, die genannten Bereiche am Kraftfahrzeug bis zu dem Maß nachgiebig zu gestalten, bis zu dem dies im Hinblick auf die Stabilität des Fahrzeugaufbaus noch vertretbar erscheint. In der Vergangenheit wurde durch die verbreitete Praxis, den Kotftügel an den Längsträger eines Fahrzeugs an einen im Wesentlichen vertikalen Flansch anzuschrauben, diese Zielvorgabe konterkariert, da der vertikale Flansch in senkrechter Richtung ausgesprochen starr wirkt.

Daneben wurde in der deutschen Patentanmeldung DE 10 2005 031 843 A1 vorgeschlagen, in der Vorderwagenstruktur eines Kraftfahrzeugs zwischen einer Kotflügelbank und einem Kotflügel ein Absorberelement anzubringen. Das in der genannten Schrift beschriebene Absorberelement ist dabei in der Art eines T- oder Doppel-T-Profils ausgeführt und weist eine Vielzahl von Stützstreben auf, die sich in etwa V-förmig zu beiden Seiten einer in Längsrichtung durchgehenden Rippe erstrecken. Diese Geometrie weist jedoch gegenüber dem bereits bekannten Stand der Technik keine hinreichende Verbesserung auf, da nach wie vor im Ergebnis der obere Kotflügelbereich zu starr in vertikaler Richtung ausgebildet bleibt.

Ein Absorberelement gemäß des Oberbegriffs des Anspruchs 1 ist in dem Dokument JP 2006 044 312 A offenbart.

Es ist daher Aufgabe der vorliegenden Erfindung, ein Absorberelement sowie ein Fahrzeug zu schaffen, das bei hinreichender Stabilität eine gegenüber dem Stand der Technik verbesserte Nachgiebigkeit und damit einen verbesserten Fußgängerschutz aufweist.

Diese Aufgabe wird gelöst durch das Absorberelement mit den in Patentanspruch 1 genannten Merkmalen sowie durch das Fahrzeug mit den in Patentanspruch 7 geführten Merkmalen. Die Unteransprüche betreffen vorteilhafte Ausführungsformen und Varianten der Erfindung.

Das erfindungsgemäße Absorberelement weist einen streifenförmigen Obergurt und einen Untergurt auf, wobei der Obergurt und der Untergurt voneinander beabstandet im Wesentlichen parallel zueinander verlaufen und miteinander über ebenfalls streifenförmige Verbindungselemente verbunden sind. Die streifenförmigen Verbindungselemente verlaufen in Längserstreckungsrichtung des Ober- bzw. Untergurts zwischen den beiden Gurten. Vorzugsweise schräg in der Art von Streben. Weiter vorzugsweise sind die streifenförmigen Verbindungselemente einstückig ausgebildet. Weiter vorzugsweise ergibt sich eine durchgängige Struktur der Verbindungselemente. Hierdurch wird bei praktisch unveränderter Stabilität der Fahrzeugstruktur eine erhöhte Toleranz gegen das Aufschlagen eines Gegenstandes von oben her sichergestellt, mit anderen Worten, es wird ein gewisses lokales Nachgeben der erfindungsgemäßen Struktur gewährleistet.

Die erfindungsgemäße Gestaltung der streifenförmigen Verbindungselemente besteht darin, dass diese im Zwischenraum zwischen dem Ober- und dem Untergurt eine wellenförmige Struktur ausbilden, die vorzugsweise durchgängig ausgebildet ist. Die Dicke der streifenförmigen Verbindungselemente kann dabei im Bereich von 1 bis 2 mm, insbesondere von 1,3 bis 1,7 mm, liegen. Die streifenförmigen Verbindungselemente können derart im Zwischenraum zwischen Obergurt und Untergurt angeordnet sein, dass diese eine in sich durchgängige wellenförmige Struktur ausbilden. Die vorzugsweise durchgängige wellenförmige Struktur ermöglicht es die bei dem Aufschlagen eines Gegenstandes von oben einwirkenden Kräfte gleichmäßig und über eine Velzahl von Kontaktpunkten aufzunehmen, zu verteilen und in der Struktur zu absorbieren. Vorzugsweise sind die streifenförmigen Verbindungselemente als ein durchgängiges Band ausgebildet. Die streifenförmigen Verbindungselemente sind aus einem polymeren Werkstoff gebildet, können aber auch aus einem metallischen Werkstoff gebildet sein.

Der Obergurt kann eine Dicke von 2 bis 4 mm, insbesondere von 2,5 bis 3,5 mm, aufweisen.

Für die streifenförmigen Verbindungselemente hat eine Wahl der Dicke in der Nähe der oberen Grenze des angegebenen Bereichs bei ca. 2 mm die Wirkung, dass die Bauhöhe des Absorberelements, also der Abstand zwischen Ober- und Untergurt, um ca. 5 mm verringert werden kann. Die Steifigkeit des Absorberelements insgesamt erhöht sich hierdurch. Allerdings wird auch bei dieser Wahl der Dicke der streifenförmigen Verbindungselemente erreicht, dass bei einem Aufprall eines Fremdkörpers Ober- und Untergurt nicht miteinander in Kontakt kommen und damit das Absorberelement seine dämpfende Wirkung verliert. Die verringerte Bauhöhe des Absorberelements führt in diesem Fall dazu, dass im Fall einer Verwendung des Absorberelements zwischen einem Kotflügel eines Fahrzeugs und einem Längsträger der Längsträger um ein geringeres Maß abgesenkt werden muss als bei Verwendung eines Absorberelements mit geringerer Wandstärke der streifenförmigen Verbindungselemente.

Um ein definiertes Verhalten des erfindungsgemäßen Absorberelements im Fall eines Schlages eines Gegenstandes von oben zu erreichen, kann es vorteilhaft sein, die streifenförmigen Verbindungselemente in ihrer Längsrichtung leicht gekrümmt auszuführen. Durch einen Aufschlag eines Gegenstandes auf das Absorberelement wird dann diese Krümmung - gegebenenfalls bis zum Bruch des Verbindungselements - verstärkt.

Eine vorteilhafte Variante der Erfindung besteht darin, dass der Obergurt an seiner den Verbindungselementen abgewandten Seite Clips zur Anbringung eines Fahrzeuganbauteils aufweist. Bei einem derartigen Fahrzeuganbauteil kann es sich beispielsweise um einen Kotflügel handeln. Die Verwendung von Clips hat dabei den Vorteil, dass während eines Lackierprozesses des Fahrzeugs Ausdehnungsunterschiede, die aufgrund der unterschiedlichen thermischen Ausdehnungskoeffizienten des Kotflügels und des Absorberelements entstehen, durch ein Aneinandergleiten der beiden verbundenen Bauteile gegeneinander abgefangen werden können. Damit ergibt sich der produktionstechnische Vorteil, dass das erfindungsgemäße Absorberelement im eingebauten Zustand im Fahrzeug beim Hersteller einem Lackierprozess unterzogen werden kann. Derartige Lackierprozesse, wie beispielsweise die kathodische Tauchlackierung, werden bei Temperaturen im Bereich von 200 °C durchgeführt. Es steht also zu erwarten, dass das erfindungsgemäße Absorberelement aufgrund der hierfür erforderlichen hohen thermischen Robustheit auch im Alltagsbetrieb eines Fahrzeugs bei den dort auftretenden Temperaturunterschieden im Bereich von 90 K nicht zu unerwünschten thermischen Effekten, wie beispielsweise Verspannungen, neigt.

Die weitere erfindungsmäße Ausführungsform der Erfindung besteht darin, dass das Absorberelement zur Verbindung mit einem Kotflügel ausgeführt ist und dass der Untergurt in seinem seitlichen Bereich in Richtung des Kotflügels verlängert und abgeknickt ist. Der abgeknickte Bereich ist dabei derart gestaltet, dass er im montierten Zustand im Bereich der Innenseite des Kotflügels im Wesentlichen parallel zu dieser verläuft. Diese Maßnahme hat die Wirkung, dass von außen seitlich auf den Kotflügel einwirkende Kräfte, wie sie beispielsweise durch Anlehnen an das Fahrzeug entstehen können, nur zu einer geringen Verringerung bzw. Änderung des Spalts zwischen Kotflügel und Motorhaube führen. Der abgeknickte Bereich nimmt einen Teil der ausgeübten Kraft auf und trägt damit wirkungsvoll zur Verformungsbegrenzung in einem derartigen Fall bei.

Zur Verschraubung des Absorberelements mit beispielsweise einem Längsträger eines Fahrzeugs kann der Untergurt Schraubenlöcher aufweisen. Dabei können diese Schraubenlöcher bei einer wellenförmigen Ausgestaltung der streifenförmigen Verbindungselemente in den "Wellentälern" angeordnet sein.

Das erfindungsgemäße Absorberelement eignet sich insbesondere zur Verwendung in einem Fahrzeug und dort im Bereich des Kotflügels. Auch die Verwendung in einem Stoßfänger eines Fahrzeugs ist denkbar.

Nachfolgend wird die Erfindung anhand der Zeichnung näher erläutert.

Es zeigen
- Figur 1: ein erfindungsgemäßes Absorberelement in einer ersten Ausführungsform der Erfindung,
- Figur 2: eine Schnittdarstellung des oberen Kotflügelbereichs eines mit dem erfindungsgemäßen Absorberelement ausgestatteten Fahrzeugs,
- Figuren 3 und 4: perspektivische Darstellungen des in den Figuren 1 bzw. 2 dargestellten Absorberelements
- Figur 5: eine Seitenansicht des erfindungsgemäßen Absorberelementes

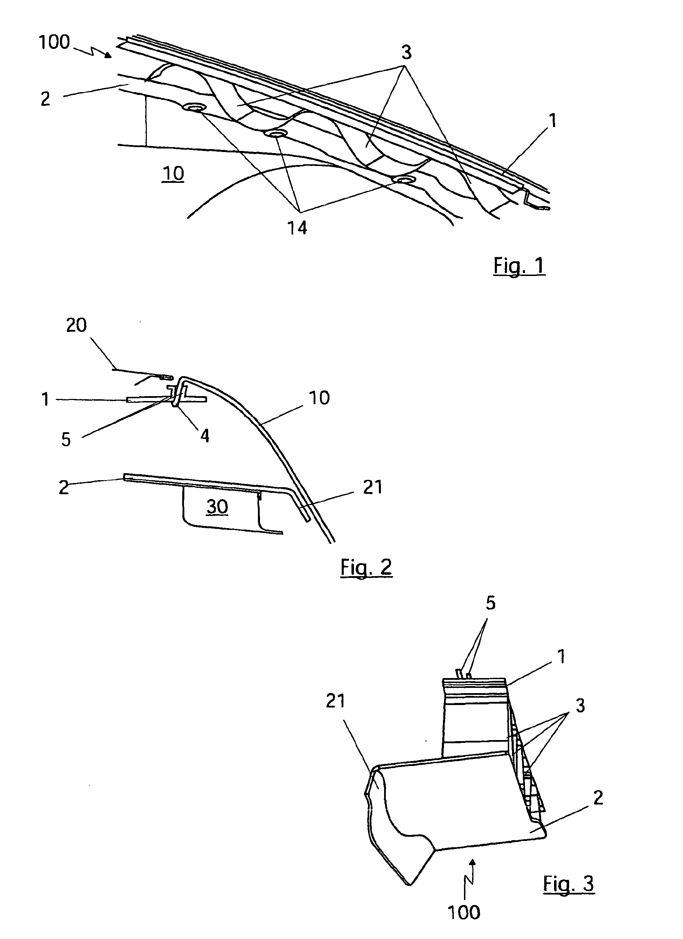

Figur 1 zeigt ein erfindungsgemäßes Absorberelement 100 in einer ersten Ausführungsform der Erfindung. Das erfindungsgemäße Absorberelement 100 wird dabei im Bereich zwischen einem nicht dargestellten Längsträger und dem oberen Teil eines Kotflügels 10 im Vorderwagenbereich eines Fahrzeugs verwendet. In der in Figur 1 dargestellten Ausführungsform der Erfindung sind der Obergurt 1 und der Untergurt 2 mittels der Verbindungselemente 3 miteinander verbunden; die Verbindungselemente 3 verlaufen dabei wellenförmig zwischen Obergurt 1 und Untergurt 2. Die durchgängig und wellenförmig ausgebildeten Verbindungselemente 3 erstrecken sich dabei in der Längsrichtung von Obergurt 1 und Untergurt 2. Der Untergurt 2 zeigt ferner die Schraubenlöcher 14, mit der das erfindungsgemäße Absorberelement an dem Längsträger des Fahrzeugs befestigt werden kann.

Figur 2 zeigt zur weiteren Verdeutlichung eine Schnittdarstellung des oberen Kotflügelbereichs eines mit dem erfindungsgemäßen Absorberelement ausgestatteten Fahrzeugs. In der Darstellung in Figur 2 ruht der Untergurt 2 auf dem Längsträger 30; der Untergurt 2 weist dabei den abgeknickten Bereich 21 auf, der abschnittsweise im Wesentlichen parallel zur Innenseite des Kotflügels 10 verläuft und diesen im Fall einer einwirkenden Querkraft zusätzlich abstützt. Der Kotflügel 10 ist mittels der Clips 4 am Obergurt 1 in einem C-Kanal 5 befestigt. Aus der Figur 2 wird erkennbar, dass die Art der Befestigung des Kotflügels 10 mittels der Clips 4 am Obergurt 1 eine Relativbewegung der beiden Fügepartner Obergurt 1 und Kotflügel 10 in einer zur Zeichenebene senkrechten Richtung zulässt, wodurch Effekte, die von unterschiedlichen thermischen Ausdehnungskoeffizienten herrühren könnten, vermindert werden. Zusätzlich in Figur 2 dargestellt ist die Motorhaube 20, deren Randbereich im Wesentlichen parallel zum Obergurt 1 verläuft.

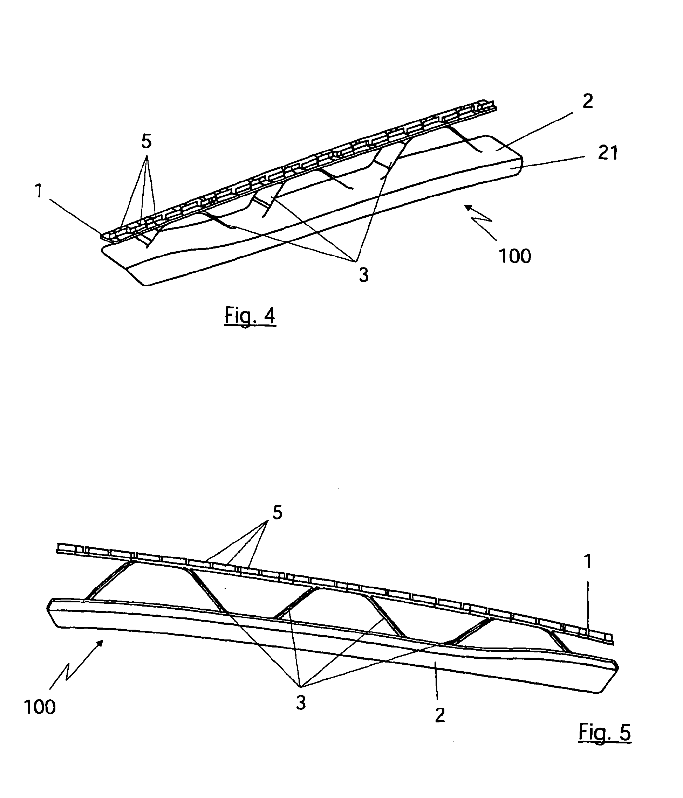

Die Figuren 3 und 4 vermitteln als perspektivische Darstellungen einen Eindruck von der dreidimensionalen Ausprägung des in den Figuren 1 bzw. 2 dargestellten Absorberelements 100. Gut erkennbar in beiden Figuren sind Obergurt 1, Untergurt 2 und die Verbindungselemente 3 sowie auf dem Obergurt 1 der C-Kanal 5 und der abgeknickte Bereich 21 am Untergurt 2.

In Figur 5 werden die Dimensionen der in den vorangegangenen Figuren dargestellten Ausführungsform der Erfindung veranschaulicht. Gut sichtbar in Figur 5 ist, dass der oben angesprochene wellenförmige Verlauf der Verbindungselemente 3 zwischen Obergurt 1 und Untergurt 2 ebenso als wechselweise an einer horizontalen Achse gespiegelte Aneinanderreihung von Trapezen betrachtet werden kann.

Obwohl sich die in den vorangegangenen Figuren dargestellten Ausführungsformen auf eine Verwendung als Absorberelement im Bereich des Vorderwagens zwischen Längsträger und Oberseite eines (Kunststoff-)Kotflügels beschränken, sei noch einmal betont, dass das erfindungsgemäße Absorberelement prinzipiell an den verschiedenen Bereichen eines Fahrzeugs, insbesondere auch im Bereich der Stoßfänger, verwendet werden kann.

## Patentansprüche

1. Absorberelement (100) für ein Fahrzeug zur Dämpfung des Aufpralls eines auf die Außenhaut des Fahrzeuges auftreffenden Körpers, wobei das Absorberelement (100) einen Obergurt (1) und einen Untergurt (2) aufweist, und wobei der Obergurt (1) und der Untergurt (2) voneinander beabstandet im Wesentlichen parallel zueinander verlaufen und miteinander über streifenförmige Verbindungselemente (3), die ebenfalls in Richtung des Obergurts (1) bzw. Untergurts (2) verlaufen, verbunden sind und das Absorberelement (100) zur Verbindung mit einem Kotflügel (10) ausgeführt ist, **dadurch gekennzeichnet, dass** der Untergurt (2) im montierlen Zustand des Absorberelements in Richtung des Kotflügels (10) verlängert und in der Weise abgeknickt ausgeführt ist, dass der abgeknickte Bereich (21) im Bereich der Innenseite des Kotflügels (10) im Wesentlichen parallel zu der Innenseite des Kotflügels verläuft und die streifenförmigen Verbindungselemente (3) derart im Zwischenraum zwischen Obergurt (1) und Untergurt (2) angeordnet sind, dass diese eine wellenförmige Struktur ausbilden.

2. Absorberelement (100) nach Anspruch 1, **dadurch gekennzeichnet, dass** die streifenförmigen Verbindungselemente (3) eine Dicke von 1 mm bis 2 mm, insbesondere von 1,3 mm bis 1,7 mm aufweisen.

3. Absorberelement (100) nach einem der vorangehenden Ansprüche 1 oder 2, **dadurch gekennzeichnet, dass** der Obergurt (1) eine Dicke von 2 mm bis 4 mm, insbesondere von 2,5 mm bis 3,5 mm aufweist.

4. Absorberelement (100) nach einem der vorangehenden Ansprüche 1 bis 3, **dadurch gekennzeichnet, dass** die streifenförmigen Verbindungselemente (3) eine Krümmung in ihrer Längsrichtung aufweisen.

5. Absorberelement (100) nach einem der vorangehenden Ansprüche 1 bis 4, **dadurch gekennzeichnet, dass** der Obergurt an seiner den Verbindungselementen abgewandten Seite einen C-Kanal (5) zur Anbringung eines Fahrzeuganbauteiles, insbesondere eines Kotflügels (10), aufweist.

6. Absorberelement (100) nach einem der vorangehenden Ansprüche 1 bis 5, **dadurch gekennzeichnet, dass** der Untergurt Schraubenlöcher (14), insbesondere zur Verschraubung des Absorberelements (100) mit einem Längsträger eines Fahrzeugs, aufweist.

7. Fahrzeug mit einem Absorberelement (100) nach einem der Ansprüche 1 bis 6.

## Claims

1. Absorber element (100) for a vehicle for damping the impact of a body striking against the outer skin of the vehicle, wherein the absorber element (100) has an upper chord (1) and a lower chord (2), and wherein the upper chord (1) and the lower chord (2) run substantially parallel to each other at a distance from each other and are connected to each other via strip-shaped connecting elements (3) which likewise run in the direction of the upper chord (1) and lower chord (2), and the absorber element (100) is designed for connection to a wing (10), **characterized in that**, in the fitted state of the absorber element, the lower chord (2) is designed such that it is extended in the direction of the wing (10) and bent down in such a manner that, in the region of the inner side of the wing (10), the bent-down region (21) runs substantially parallel to the inner side of the wing, and the strip-shaped connecting elements (3) are arranged in the intermediate space between the upper chord (1) and lower chord (2) in such a manner that said connection elements form an undulatory structure.

2. Absorber element (100) according to Claim 1, **characterized in that** the strip-shaped connecting elements (3) have a thickness of 1 mm to 2 mm, in particular of 1.3 mm to 1.7 mm.

3. Absorber element (100) according to either of the preceding Claims 1 and 2, **characterized in that** the upper chord (1) has a thickness of 2 mm to 4 mm, in particular of 2.5 mm to 3.5 mm.

4. Absorber element (100) according to one of the preceding Claims 1 to 3, **characterized in that** the strip-shaped connecting elements (3) have a curvature in the longitudinal direction thereof.

5. Absorber element (100) according to one of the preceding Claims 1 to 4, **characterized in that** that side of the upper chord which faces away from the connecting elements has a C channel (5) for attaching a vehicle add-on part, in particular a wing (10).

6. Absorber element (100) according to one of the preceding Claims 1 to 5, **characterized in that** the lower chord has screw holes (14), in particular for screwing the absorber element (100) to a longitudinal member of a vehicle.

7. Vehicle with an absorber element (100) according to one of Claims 1 to 6.

## Revendications

1. Élément absorbeur (100) de véhicule servant à amortir le choc provoqué par un corps rencontrant la carrosserie extérieure du véhicule, l'élément absorbeur (100) comportant une sangle supérieure (1) et une sangle inférieure (2) et la sangle supérieure (1) et la sangle inférieure (2) s'étendant pour l'essentiel parallèlement l'une par rapport à l'autre à une certaine distance l'une de l'autre et étant reliées l'une à l'autre par le biais d'éléments de liaison (3) en forme de bande s'étendant également en direction de la sangle supérieure (1) et/ou de la sangle inférieure (2) et l'élément absorbeur (100) étant conçu pour pouvoir être relié à une aile (10), **caractérisé en ce que** la sangle inférieure (2) est allongée dans la direction de l'aile (10) à l'état monté de l'élément absorbeur et courbée de façon à ce que la zone courbée (21) s'étende pour l'essentiel parallèlement au côté intérieur de l'aile dans la zone du côté intérieur de l'aile (10) et que les éléments de liaison (3) en forme de bande soient disposés de telle sorte dans l'espace intermédiaire prévu entre la sangle supérieure (1) et la sangle inférieure (2) que ceux-ci forment une structure en forme d'arbre.

2. Élément absorbeur (100) selon la revendication 1, **caractérisé en ce que** l'épaisseur des éléments de liaison (3) en forme d'arbre va d'1 mm à 2 mm, notamment d'1,3 mm à 1,7 mm.

3. Élément absorbeur (100) selon l'une quelconque des revendications 1 ou 2, **caractérisé en ce que** l'épaisseur de la sangle supérieure (1) va de 2 mm à 4 mm, notamment de 2,5 mm à 3,5 mm.

4. Élément absorbeur (100) selon l'une quelconque des revendications 1 à 3, **caractérisé en ce que** les éléments de liaison (3) en forme de bande sont courbés dans leur direction longitudinale.

5. Élément absorbeur (100) selon l'une quelconque des revendications 1 à 4, **caractérisé en ce que** la sangle supérieure comporte, au niveau de son côté opposé aux éléments de liaison, un canal en C (5) servant à appliquer dessus un composant du véhicule, notamment une aile (10).

6. Élément absorbeur (100) selon l'une quelconque des revendications 1 à 5, **caractérisé en ce que** la sangle inférieure comporte des trous de vis (14) servant notamment à raccorder par vissage l'élément absorbeur (100) à un montant longitudinal d'un véhicule.

7. Véhicule équipé d'un élément absorbeur (100) selon l'une quelconque des revendications 1 à 6.
